(19) Europäisches Patentamt
European Patent Office
Office européen des brevets

(11) Veröffentlichungsnummer: **0 447 977 A1**

# (12) EUROPÄISCHE PATENTANMELDUNG

(21) Anmeldenummer: **91104021.0**

(22) Anmeldetag: **15.03.91**

(51) Int. Cl.[5]: **C07C 39/17**, C07C 37/20, C07C 37/52

(30) Priorität: **22.03.90 DD 338955**

(43) Veröffentlichungstag der Anmeldung: **25.09.91 Patentblatt 91/39**

(84) Benannte Vertragsstaaten: **CH DE FR GB IT LI**

(71) Anmelder: **LEUNA-WERKE AG**
**O-4220 Leuna 3(DE)**

(72) Erfinder: **Görmar, Gerhard, Dr.**
**Amalienstrasse 7**
**O-7031 Leipzig(DE)**
Erfinder: **Seiffarth, Klaus, Dr.**
**Block 032/06**
**O-4090 Halle-Neustadt(DE)**
Erfinder: **Schulz, Manfred, Prof. Dr.**
**Friesenstrasse 16**
**O-4200 Merseburg(DE)**

## (54) Verfahren zur Herstellung von p-Alkylcalix[4]arenen.

(57) Ziel ist eine einfache und wirtschaftliche Herstellung von p-Alkylcalix[4]arenen der allgemeinen Formel I

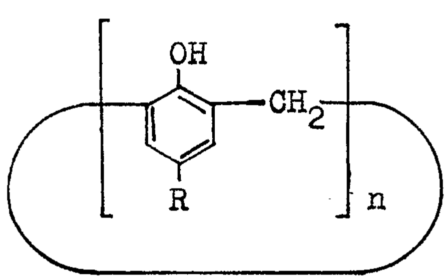

I

mit n = 4.
Die Verbindungen werden hergestellt aus einem Vorkondensat, hergestellt aus p-Alkylphenol und Formaldehyd, oder aus p-tert.-Alkylcalix[8]arenen oder aus einem Gemisch aus p-Alkylphenol und Paraformaldehyd, jeweils in Gegenwart von Alkalihydroxiden als Katalysator, in einem Gemisch geradkettiger Kohlenwasserstoffe der Kettenlänge $C_{11}$ bis $C_{20}$ bei 217 bis 257 °C. Die p-Alkylcalix[4]arene werden in gut kristallisierter Form direkt aus dem Reaktionsansatz isoliert. Besonders geeignet ist das Verfahren zur Herstellung von p-tert.-Butylcalix[4]-aren (Formel I, R = t.-$C_4H_9$, n = 4) und p-Tert.-Amylcalix[4]aren (Formel I, R = t.-$C_5H_{11}$, n = 4).
Die erfindungsgemäß hergestellten Verbindungen können zur Metallionenextraktion bzw. für ionensensitive Elektroden, als Stabilisatoren von polymeren Werkstoffen sowie als Syntheseausgangsstoff für Calix[4] arenderivate eingesetzt werden.

EP 0 447 977 A1

Die Erfindung betrifft ein Verfahren zur Herstellung von p-Alkylcalix[4]arenen, die als Stabilisatoren für Polymere oder zur Herstellung ionensensitiver Elektroden oder als macrocyclische Synthesebausteine verwendbar sind.

p-Alkylcalix [4]arene haben auf Grund ihrer besonderen Struktur in den letzten Jahren zunehmend an Interesse gewonnen (Top. Curr. Chem. 123 (1984) 1).

Für einen praktischen Einsatz der p-Alkylcalix[4]arene macht es sich erforderlich, technologisch einfache und wirtschaftliche Herstellungsverfahren für diese Verbindungen zu entwickeln. Gemäß Literatur (J. Org. Chem. 51(1986) 742) wird zur Herstellung der p-Alkylcalix[4]arene durch basenkatalysierte Reaktion der polare Diphenylether als Reaktionsmedium eingesetzt. Der Nachteil dieses Verfahrens besteht darin, daß neben einer erheblichen Geruchsbelästigung durch den Diphenylether, dieser als aromatische Verbindung toxisch bedenklich ist. Außerdem ist eine aufwendige Stufe zur Isolierung der p-Alkylcalix[4]arene bei diesem Verfahren notwendig. Weiterhin können p-Alkylcalix[4]arene durch basenkatalysierte Reaktion von p-Alkylphenolen und Paraformaldehyd in Tetralin hergestellt werden (J. Org. Chem. 43 (1978) 4280).

Das Tetralin als Reaktionsmedium hat den Nachteil, daß es als aromatische Verbindung toxisch ist und daß es auf Grund seiner leichten Autoxidierbarkeit Peroxide bildet, die durch ihre spontane Zersetzung bei höheren Temperaturen zu erheblichen Gefährdungen führen. Außerdem wird bei diesem Verfahren Kalium-tert.-butylat als Katalysator angewendet, das nur in einem aufwendigen Verfahren aus Kalium und tert.-Butanol erhalten wird und für eine technische Anwendung zu teuer ist.

Der Erfindung liegt das Problem zugrunde, ein technologisch einfaches und wirtschaftliches Verfahren zur Herstellung von p-Alkylcalix[4]arenen zu entwickeln, wobei die Herstellung unter Verwendung eines nichttoxischen und geruchsfreien Reaktionsmediums in Gegenwart leicht verfügbarer basischer Katalysatoren erfolgen soll.

Das Problem wird durch ein Verfahren zur Herstellung von p-Alkylcalix[4]arenen der allgemeinen Formel I

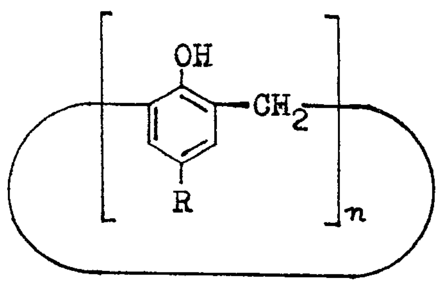

I

gelöst, in welcher

R = n-Alkyl $C_1$ bis $C_4$; iso-Alkyl $C_3$ bis $C_5$; tert.-Alkyl $C_4$ und $C_5$; Cyclohexyl und

n = 4

bedeuten, aus einem Vorkondensat, hergestellt aus dem entsprechenden Alkylphenol der allgemeinen Formel II

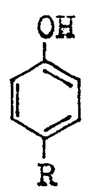

II,

in der R die gleiche Bedeutung wie in Formel I hat, und Formaldehyd in Form einer wäßrigen Lösung mit vorzugsweise 30 bis 40 Masseanteilen in % Formaldehyd, oder aus einem p-Alkylcalix[8]aren der allgemeinen Formel I mit

R wie in Formel I und

n = 8,

oder aus einem Gemisch aus dem p-Alkylphenol der allgemeinen Formel II mit

R wie in Formel I
und Paraformaldehyd durch eine mit Alkalihydroxid katalysierte Reaktion in einem organischen Lösungsmittel als Reaktionsmedium, indem erfindungsgemäß als organisches Lösungsmittel ein Gemisch geradkettiger Kohlenwasserstoffe der Kettenlänge $C_{11}$ bis $C_{20}$ und des Siedebereiches 250 bis 260 °C bei Normaldruck eingesetzt wird, in welches man den jeweiligen Ausgangsstoff und das Alkalihydroxid einbringt, 1 bis 10 Stunden bei 217 bis 257 °C unter Sauerstoffausschluß rührt, dabei das entstehende Reaktionswasser abdestilliert, anschließend unumgesetzten Feststoff bei 100 bis 160 °C aus dem Reaktionsgemisch abtrennt, die feststoffreiche Reaktionslösung auf 17 bis 42 °C abkühlt und das ausgefallene, gut kristalline p-Alkylcalix[4]aren in an sich bekannter Weise isoliert, mit einem Kohlenwasserstoffgemisch der Kettenlänge $C_5$ bis $C_7$ wäscht und im Vakuum bei 0,5 bis 1,0 kPa trocknet.

Als Gemisch geradkettiger Kohlenwasserstoffe der Kettenlänge $C_{11}$ bis $C_{20}$ wird ein durch Molsiebextraktion aus einem Erdölmitteldestillatschnitt isoliertes n-Paraffingemisch der Zusammensetzung 2,5 bis 10 Masseteile in % n-Paraffine $C_{11}$ bis $C_{12}$, 80 bis 90 Masseanteile in % n-Paraffine $C_{13}$ bis $C_{17}$ und 0,5 bis 10 Masseanteile in % n-Paraffine $C_{18}$ bid $C_{20}$ eingesetzt. Als Alkalihydroxid wird vorzugsweise Kaliumhydroxid bzw. Natriumhydroxid in einem Verhältnis von 2 bis 10 Stoffmengenanteilen in % bezogen auf verwendete phenolische Einheit eingesetzt. Der Einsatz erfolgt vorzugsweise in Form einer wäßrigen Lösung.

Nach dem erfindungsgemäßen Verfahren werden überraschenderweise p-Alkylcalix[4]arene in gut kristallisierter Form unter Verwendung eines fast geruchlosen, nichttoxischen Reaktionsmediums erhalten. Verbindungen, die vorzugsweise durch dieses erfindungsgemäße Verfahren hergestellt werden, sind das p-tert.-Butylcalix[4]aren (Formel I, n = 4, R = t.-$C_4H_9$) und p-tert.-Amylcalix[4]aren (Formel I, n = 4, R = t.-$C_5H_{11}$). Die nach dem erfindungsgemäßen Verfahren hergestellten p-Alkylcalix[4]arene können zur Metallionenextraktion bzw. für ionensensitive Elektroden, als Stabilisatoren von polymeren Werkstoffen sowie als Syntheseausgangsstoff für Calix[4]arenderivate eingesetzt werden.

Nachfolgend wird die Erfindung an einigen Beispielen erläutert, ohne sie dadurch einzuschränken.

Beispiel 1

50 g p-tert.-Butylphenol, 70cm$^3$ 30%ige wäßrige Formaldehydlösung und 0,6 g Natriumhydroxid werden in einem offenen Kolben eine Stunde 100 bis 110 °C ausgesetzt. Die erhaltene hochviskose Masse läßt man auf Raumtemperatur abkühlen und zerkleinert sie nach dem Erstarren in kleine Stücke. Diese werden in einem Rührgefäß mit Wasserabscheider in 400 cm$^3$ eines Gemisches geradkettiger Paraffine $C_{11}$ bis $C_{20}$ suspendiert und unter Rühren und Sauerstoffausschluß erwärmt. Bei 110 °C beginnt das Abdestillieren des Reaktionswassers. Die Temperatur wird weiter gesteigert, wobei oberhalb 152 °C ein weißer Feststoff zunächst ausfällt. Anschließend wird bei 217 bis 252 °C etwa 5 Stunden zum Sieden erhitzt. Dabei geht der überwiegende Teil des Feststoffes wieder in Lösung.

Nach den 5 Stunden Reaktionszeit läßt man den verbleibenden Feststoff sich absetzen und zieht bei 100 bis 160 °C die heiße Lösung vom abgesetzten Feststoff ab. Beim Abkühlen der Lösung auf Raumtemperatur scheiden sich Kristalle des p-tert.-Butylcalix[4]arens aus, die durch Filtration isoliert und mit einem Gemisch von Paraffinen $C_5$ bis $C_7$ gewaschen werden. Anschließend wird im Vakuum bei 0,5 bis 1,0 kPa getrocknet.
Ausbeute: 32 g (60% bezogen auf Phenol); Fp. 340 bis 345 °C.

Beispiel 2

50 g p-tert.-Butylcalix[8]aren (Formel I, n = 8, R = tert.-Butyl) und 0,6 g Natriumhydroxid in 2 ml Wasser werden in einem Rührgefäß mit Wasserabscheider in 400 cm$^3$ eines Gemisches geradkettiger Paraffine der vorzugsweisen Kettenlänge $C_{13}$ bis $C_{17}$ suspendiert und unter Sauerstoffausschluß 6 Stunden bei 217 bis 252 °C gerührt. Dabei geht der überwiegende Teil des Feststoffs in Lösung. Nach den 6 Stunden läßt man eventuell unumgesetztes p-tert.-Butylcalix[8]aren sich absetzen und zieht die heiße flüssige Phase vom Feststoff ab. Beim Abkühlen der flüssigen Phase auf Raumtemperatur scheiden sich Kristalle aus, die durch Filtration isoliert und mit einem leichtsiedenden Kohlenwasserstoffgemisch $C_5$ bis $C_7$ gewaschen werden. Anschließend erfolgt die Trocknung im Vakuum bei 0,5 bis 1,0 kPa.
Ausbeute: 35 g (70 % bezogen auf t.-Butylcalix[8]aren); Fp. 341 bis 346 °C.

Beispiel 3

In einem Rührgefäß mit Wasserabscheider werden 50 g p-tert.-Butylphenol, 20 g Paraformaldehyd und

0,8 $cm^3$ 10 n wäßrige Kaliumhydroxidlösung in 500 $cm^3$ eines Gemisches geradkettiger Paraffine unter Sauerstoffausschluß zwei Stunden auf 120 °C erwärmt und anschließend vier Stunden bei 247 °C zum Sieden erhitzt.

Den nicht umgesetzten Feststoff läßt man sich absetzen und trennt die heiße Reaktionslösung von Feststoff ab. Beim Abkühlen auf 17 bis 27 °C kristallisiert p-tert.-Butylcalix[4]aren aus, das isoliert und mit einem Gemisch von Paraffinen $C_5$ bis $C_7$ gewaschen wird. Anschließend trocknet man es im Vakuum bei 0,5 bis 1,0 kPa.
Ausbeute: 17 g (33 % bezogen auf Phenol); Fp. 340 bis 345 °C.

Beispiel 4

Wie im Beispiel 1 beschrieben, wird aus 50 g tert.-Amylphenol, 70 $cm^3$ einer 30%igen wäßrigen Formaldehydlösung und 0,6 g Natriumhydroxid ein Vorkondensat hergestellt. Dieses wird ebenfalls wie im Beispiel 1 beschrieben in einem Rührgefäß in 400 $cm^3$ eines Gemisches geradkettiger Paraffine suspendiert und weiterverarbeitet.
Ausbeute: 22 g (50 % bezogen auf eingesetztes Phenol), Fp. 308 bis 310 °C.

**Patentansprüche**

**1.** Verfahren zur Herstellung von p-Alkylcalix[4]arenen der allgemeinen Formel

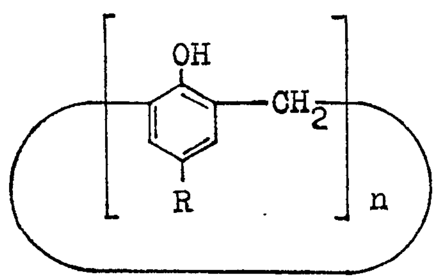

I,

in welcher R = n-Alkyl $C_1$ bis $C_4$; iso-Alkyl $C_3$ bis $C_5$; tert.-Alkyl $C_4$ und $C_5$ bzw. Cyclohexyl und n = 4 bedeuten,

- aus einem Vorkondensat, hergestellt aus dem entsprechenden p-Alkylphenol der allgemeinen Formel

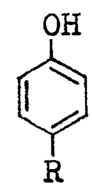

II,

in der R die gleiche Bedeutung wie in Formel I hat, und Formaldehyd in Form einer wäßrigen Lösung, vorzugsweise mit 30 bis 40 Masseanteilen in % Formaldehyd,
- oder aus einem p-Alkylcalix[8]aren der allgemeinen Formel I mit R wie in Formel I und n = 8
- oder aus einem Gemisch aus dem p-Alkylphenol der allgemeinen Formel II, in der R die gleiche Bedeutung wie in Formel I hat, und Paraformaldehyd

durch eine mit Alkalihydroxid katalysierte Reaktion in einem organischen Lösungsmittel als Reaktionsmedium, dadurch gekennzeichnet, daß als organisches Lösungsmittel ein Gemisch geradkettiger Kohlenwasserstoffe der Kettenlänge $C_{11}$ bis $C_{20}$ und des Siedebereiches 250 bis 260 °C bei Normaldruck eingesetzt wird, in welches man den jeweiligen Ausgangsstoff und das Alkalihydroxid einbringt, 1 bis 10 Stunden bei 217 bis 257 °C unter Sauerstoffausschluß rührt, dabei das entstehende Reaktionswasser abdestilliert, anschließend unumgesetzten Feststoff bei 100 bis 160 °C aus dem Reaktionsgemisch abtrennt, die feststoffreie Reaktionslösung auf 17 bis 42 °C abkühlt und das

ausgefallene, gut kristalline p-Alkylcalix[4]aren in an sich bekannter Weise isoliert, mit einem Kohlenwasserstoffgemisch der Kettenlänge $C_5$ bis $C_7$ wäscht und im Vakuum bei 0,5 bis 1,0 kPa trocknet.

2. Verfahren nach Anspruch 1, dadurch gekennzeichnet, daß als Gemisch geradkettiger Kohlenwasserstoffe der Kettenlänge $C_{11}$ bis $C_{20}$ ein durch Molsiebextraktion aus einem Erdölmitteldestillatschnitt isoliertes n-Paraffingemisch der Zusammensetzung 2,5 bis 1o Masseanteile in % n-Paraffine $C_{11}$ bis $C_{12}$, 80 bis 90 Masseanteile in % n-Paraffine $C_{13}$ bis $C_{17}$ und 0,5 bis 10 Masseanteile in % n-Paraffine $C_{18}$ bis $C_{20}$ eingesetzt wird.

3. Verfahren nach Anspruch 1, dadurch gekennzeichnet, daß als Alkalihydroxid Natrium- oder Kaliumhydroxid, vorzugsweise in Form einer wäßrigen Lösung in einem Verhältnis von 2 bis 10 Stoffmengenanteilen in % bezogen auf verwendete phenolische Einheit eingesetzt wird.

Europäisches Patentamt

# EUROPÄISCHER RECHERCHENBERICHT

Nummer der Anmeldung

**EP 91 10 4021**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | Topics in Current Chemistry vol. 123, 1984, Springer Verlag, Berlin, D Seiten 1 - 47; C.D. Gutsche: "The Calixarenes"<br>* Seite 4, Zeile 27; Seite 7, Zeilen 33-37 *<br>- - - | 1 | C 07 C 39/17<br>C 07 C 37/20<br>C 07 C 37/52 |
| A | PATENT ABSTRACTS OF JAPAN vol. 8, no. 218 (C-245)(1655) 04 Oktober 1984,<br>& JP-A-59 104333 (HITACHI KASEI) 16 Juni 1984,<br>* das ganze Dokument *<br>- - - | 1 | |
| A | US-A-3 677 986 (A.D. BUCHANAN)<br>* Spalte 7, Zeile 64 - Spalte 8, Zeile 16; Anspruch 1 *<br>- - - - - | 1 | |
| | | | RECHERCHIERTE SACHGEBIETE (Int. Cl.5) |
| | | | C 07 C |

Der vorliegende Recherchenbericht wurde für alle Patentansprüche erstellt

| Recherchenort | Abschlußdatum der Recherche | Prüfer |
|---|---|---|
| Berlin | 03 Juli 91 | PROBERT C.L. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument